Europäisches Patentamt

European Patent Office

Office européen des brevets

(11) Publication number: **0 399 589**
**A1**

(12) **EUROPEAN PATENT APPLICATION**

(21) Application number: **90201198.0**

(22) Date of filing: **11.05.90**

(51) Int. Cl.5: **C12P 41/00, C12P 13/00**

(30) Priority: **18.05.89 GB 8911456**

(43) Date of publication of application:
**28.11.90 Bulletin 90/48**

(84) Designated Contracting States:
**AT BE CH DE DK ES FR GB GR IT LI LU NL SE**

(71) Applicant: **SHELL INTERNATIONALE
RESEARCH MAATSCHAPPIJ B.V.
Carel van Bylandtlaan 30
NL-2596 HR Den Haag(NL)**

(72) Inventor: **Phillips, Gareth Thomas
5 The Finches
Sittingbourne, Kent(GB)**
Inventor: **Shears, Jemery Hallam
3 The Mead, Leybourne
Maidstone, Kent(GB)**

(74) Representative: **Bennett, David Arthur Horder
et al
4, York Road
London SE1 7NA(GB)**

(54) **Preparation of n-acyl alkylamines.**

(57) N-Acyl-1-methyl-omega-phenylalkylamines predominantly in the form of the R enantiomer, are prepared by a process using a biocatalyst capable of stereoselectively hydrolysing the N-acyl group of the S enantiomer and recovering the remaining N-acyl-1-methyl-omega-phenylalkylamine predominantly in the form of the R enantiomer. The S enantiomer may be hydrolysed to yield the S enantiomer of the corresponding amine, which may be recovered.

EP 0 399 589 A1

## PREPARATION OF N-ACYL ALKYLAMINES

The present invention relates to a process for preparing certain (R)-N-acyl-1-methyl-omega-phenylalkylamines. Such compounds find use as intermediates in the preparation of pharmaceuticals.

N-protected (R)-1-methyl-omega-phenylalkylamines find use as intermediates in the preparation of a number of pharmaceuticals. One particular pharmaceutical is the compound dilevalol, namely (R)-1-hydroxy-2-[(R)-(1-methyl-3-phenylpropyl) amino]ethyl salicylamide, also known as (R,R)-labetalol. Dilevalol is an antihypertensive pharmaceutical. It is described in European patent application publication number 009702.

According to European patent application publication number 009702, dilevalol may be prepared from an N-protected (R)-1-methyl-3-phenylpropylamine in a multistep process which comprises reacting the protected amine with an 0-protected 4-hydroxy-alpha-bromo-3-carbamoylacetophenone; reducing the product to afford a mixture of isomers, and separating from this mixture an N,O-protected (R)-1-hydroxy-2-[(R)-(1-methyl-3-phenylpropyl)amino]ethyl] salicylamide; and removing the protecting groups to afford dilevalol, or a pharmaceutically acceptable salt thereof. The N-protected (R)-1-methyl-3-phenylpropylamine used in the process is prepared by resolving racemic N-protected-1-methyl-3-phenylpropylamine. An example of an N-protected-1-methyl-3-phenylpropylamine given in the specification is the N-benzyl derivative which may be prepared either by reaction of benzylamine with benzyl acetone followed by reduction of the resulting Schiff's base, or by benzylation of racemic 1-methyl-3-phenylpropylamine.

The preparation of the R enantiomers of N-protected 1-methyl-3-phenylpropylamines is particularly expensive, and many attempts have been made to find improved processes for preparing them.

In one attempt, described in United States patent number 4,658,060, the N-protecting group is an optically active 1-phenylethyl group. The R enantiomer is prepared by reacting (R)-alpha-methylbenzylamine with benzylacetone to give enamines which are reduced to afford a mixture of isomers from which the desired (R,R)-diastereoisomer may be separated by conventional physical methods. A disadvantage of this process has been that an optically active starting material, namely (R)-alpha-methylbenzylamine, is required.

In another approach, described in Japanese patent application publication number JP 63054351, (R)-1-methyl-3-phenylpropylamine is prepared by reduction of the corresponding R azide. This azide is prepared by a multistep process, which comprises the asymmetric reduction of benzylacetone with a microorganism to give (S)-1-methyl-3-phenylpropanol; conversion of this alcohol into the corresponding (S)-p-toluenesulphonate; and reaction of this product with a metal azide to afford the desired (R)-1-methyl-3-phenylpropylazide. However, this process is unattractive because of the large number of steps it involves.

In yet another attempt, described in Japanese patent application publication number JP 63237796, (R)-1-methyl-3-phenylpropylamine is prepared from the racemic material using a microorganism capable of preferentially metabolising the S enantiomer.

More generally, the preparation of R and S enantiomers of a range of N-p-aminophenylacetylamino derivatives has been attempted by Rossi et al (J. Org. Chem., Vol. 44, No. 13, 1979, pages 2222 to 2225). The enantiomers were selectively prepared using benzylpenicillinacylase as biocatalyst. In particular, Rossi et al prepared the R enantiomers of N-p-amino-1-phenylacetyl-1-phenylethylamine and N-p-amino-1-phenylacetyl-1-phenyl-n-propylamine, in yields of 19% and 58% optical purity respectively from the corresponding racemate.

However, the biocatalyst benzylpenicillinacylase has been found to be specific in its activity, being active only with respect to N-acyl-1-methyl-omega-phenylalkylamines in which the acyl group is a phenylacetyl derivative. In the commercial application of such a process, it is highly desirable to be able to select the N-protecting group from a range of structurally simple groups, as such groups are eventually removed and lost in the preparation of the pharmaceutical end products.

Most surprisingly, a novel and advantageous process has now been found for preparing certain (R)-N-acyl-1-methyl-omega-phenylalkylamines which involves the use of a biocatalyst.

Accordingly, the present invention provides a process for the preparation of an N-acyl-1-methyl-omega-phenylalkylamine in which the acyl group is an aliphatic acyl group and the alkyl group has from 1 to 3 carbon atoms, predominantly in the form of the R enantiomer, which comprises supplying N-acyl-1-methyl-omega-phenylalkylamine to a biocatalyst capable of stereoselectively hydrolysing the N-acyl group of the S enantiomer and recovering the remaining N-acyl-1-methyl-omega-phenylalkylamine predominantly in the form of the R enantiomer.

It has been found that (R)-N-acyl-1-methyl-omega-phenylalkylamine having an enantiomeric purity in excess of 99.5% may be prepared in high yield by the process according to the invention. Enantiomeric purity is defined as 100% [R enantiomer]/([R enantiomer] + [S enantiomer]).

The N-acyl-1-methyl-omega-phenylalkylamine is preferably supplied to the biocatalyst in the form of a racemic mixture.

The acyl group in the N-acyl-1-methyl-omega-phenylalkylamine is preferably an alkanoyl group, alkoxycarbonyl group or formyl. Alkanoyl groups preferably have from 2 to 6 carbon atoms, more preferably from 2 to 4 carbon atoms, with acetyl being especially preferred. Alkoxycarbonyl groups preferably have from 2 to 6 carbon atoms, more preferably from 2 to 4 carbon atoms, with methoxycarbonyl being especially preferred. The acyl group is most preferably acetyl.

The alkyl group in the N-acyl-1-methyl-omega-phenylalkylamine has from 1 to 3 carbon atoms. The alkyl group is preferably propyl.

The biocatalyst may be a microorganism, in particular a bacterium, fungus or yeast, or a plant or animal cell, or an extract thereof, or an enzyme. Preferably the biocatalyst is a microorganism, an extract thereof, or an enzyme. More preferably the biocatalyst is a microorganism selected from the genera Rhodococcus, Bacillus, Arthrobacter and Corynebacterium.

Specific examples of microorganisms useful as biocatalyst in the process according to the invention are Corynebacterium pseudodiphtheriticum NCIMB 40145, Rhodococcus sp NCIMB 40139; Arthrobacter sp NCIMB 40140; Arthrobacter sp NCIMB 40141; Corynebacterium sp NCIMB 40142; Corynebacterium sp NCIMB 40143 and Corynebacterium sp NCIMB 40144, all deposited on 9th May, 1989 at the National Collections of Industrial and Marine Bacteria Ltd; Torry Research Station, P.O. Box 31, 135 Abbey Road, Aberdeen AB9 8DG, United Kingdom. Further specific examples of microorganisms useful as biocatalyst in the process are Bacillus sp NCIMB 40245 and Bacillus sp NCIMB 40246, both deposited on 21st December, 1989 at the National Collections of Industrial and Marine Bacteria Ltd, Torry Research Station, P.O. Box 31, 135 Abbey Road, Aberdeen AB9 8DG, United Kingdom.

Microorganisms suitable for use in the process according to the invention have been isolated from soil samples by a selection procedure which identifies those microorganisms that are capable of growing on acetamides, for example N-sec-butylacetamide.

When a microorganism is used as the biocatalyst, it may be in a growing or non-growing state.

While the hydrolysis of N-acyl-1-methyl-omega-phenylalkylamine is usually carried out in the presence of whole microorganism cells, the hydrolytic enzyme system may have been completely or partly extracted from the microorganism prior to carrying out the hydrolysis. To avoid unnecessary separation, enzyme purification and enzyme immobilisation procedures, the enzyme is usually present with at least some of the cell components. When in association with the cells, these may be live or dead, intact, treated in some way or themselves immobilised and optionally homogenised, so long as the enzyme component itself is retained in an active and stable form to allow the hydrolysis to proceed. Immobilisation of the enzyme or the cells may be by any of the methods known in the art so long as the enzyme hydrolysing activity is retained intact.

The microorganisms are preferably cultured prior to use for the hydrolysis for about 1 to 10 days, whereafter the cells are suspended in a liquid salts medium, preferably a minimal liquid nutrient medium, and N-acyl-1-methyl-omega-phenylalkylamine is subjected to the action of the cells. To grow the microorganisms used for the hydrolysis, ordinary culture media containing an assimilable carbon source (for example glucose, lactate, sucrose, etc.), a nitrogen source (for example ammonium sulphate, ammonium nitrate, ammonium chloride, etc.), with an agent for an organic nutrient source (for example yeast extract, malt extract peptone, meat extract, etc.) and an inorganic nutrient source (for example phosphate, magnesium, potassium, zinc, iron and other metals in trace amounts) may be used, along with an acetamide inducer (such as acetamide or an N-alkyl acetamide, e.g. N-sec-butylacetamide) or, preferably a thioacetamide (such as thioacetanilide) as necessary. As an alternative culture medium, a medium containing salts plus an acetamide (for example N-sec-butylacetamide) or thioacetamide is used.

A temperature between 0 and 45°C and a pH between 3.5 and 8 is maintained during the growth of the microorganisms.

Preferably the microorganisms are grown at a temperature between 20 and 37°C and at a pH between 4 and 8.

During the hydrolysis of N-acyl-1-methyl-omega-phenylalkylamine, an ordinary culture medium may be used containing an assimilable carbon source when required (for example glucose, lactate, sucrose, fructose, glycerol etc.), a nitrogen source when required (for example ammonium sulphate, ammonium nitrate, ammonium chloride, etc.), with an agent for an organic nutrient source when required (for example yeast extract, salt extract, peptone, meat extract, etc.) and an inorganic nutrient source when required (for example phosphate, magnesium, potassium, zinc, iron and other metals in trace amounts). Preferably during the hydrolysis of N-acyl-1-methyl-omega-phenylalkylamine the microorganisms are held in a substantially non-growing state using a minimal culture medium. The microorganisms can be used in the non-growing state for example under exclusion of the assimilable carbon source or under exclusion of the nitrogen source in an aqueous medium containing salts (for example sodium chloride, potassium phosphate etc), or in water alone. A temperature between 0 and 45°C and a pH between 3.5 and 9 is maintained during this stage. Preferably the microorganisms are kept at a temperature between 20 and 37°C and a pH between 4 and 8.

The N-acyl-1-methyl-omega-phenylalkylamine can conveniently be supplied to the biocatalyst at concentrations of from 1 to 200g/l, preferably at a concentration of at least 5g/l.

The remaining N-acyl-1-methyl-omega-phenylalkylamine predominantly in the form of the R enantiomer, can be recovered and purified according to any of the procedures known per se for such products, for example by fractional crystallisation from the broth or differential acid/base/solvent extraction.

In addition to preparing the R enantiomer of N-acyl-1-methyl-omega-phenylalkylamine, the process of the present invention may also be used to prepare the S enantiomer of the corresponding 1-methyl-omega-phenylalkylamine. In the process, the S enantiomer of N-acyl-1-methyl-omega-phenylalkylamine is selectively hydrolysed by the biocatalyst to leave the desired R enantiomer. By appropriate selection of the biocatalyst for use in the process, the hydrolysis of the (S)-N-acyl-1-methyl-omega-phenylalkylamine can yield the corresponding (S)-1-methyl-omega-phenylalkylamine. (S)-1-methyl-omega-phenylalkylamine may be recovered and purified according to any of the procedures known per se for such products.

The N-acyl-1-methyl-omega-phenylalkylamine predominantly in the form of the R enantiomer prepared by the process of the present invention may be used in the preparation of the corresponding 1-methyl-omega-phenylalkylamine predominantly in the form of the R enantiomer, a salt thereof or an N-protected form thereof.

The N-acyl-1-methyl-omega-phenylalkylamine may be converted into the 1-methyl-omega-phenylalkylamine by hydrolysis either with a base such as an alkali metal hydroxide, or an acid such as an inorganic acid, for example hydrochloric acid or sulphuric acid. The hydrolysis is effected in the presence of water, conveniently at a temperature of from 50°C up to the reflux temperature.

The 1-methyl-omega-phenylalkylamine may be converted into a salt by conventional methods, for example by treatment with a hydrohalic acid, to afford a hydrohalide salt, for example the hydrochloride or hydrobromide.

Other N-protected forms of the 1-methyl-omega-phenylalkylamine may also be prepared using conventional methods. For example, N-aralkyl forms such as N-benzyl or N-alpha-methylbenzyl may be prepared by reaction of the amine with the appropriate aralkyl halide.

The R enantiomer of N-acyl-1-methyl-omega-phenylalkylamine find use as intermediates in the preparation of compounds such as pharmaceuticals. In particular, N-acyl-1-methyl-3-phenylpropylamine predominantly in the form of the R enantiomer is of use in the preparation of (R)-1-hydroxy-2-[(R)-(1-methyl-3-phenylpropyl)-amino] ethyl salicylamide, or a pharmaceutically acceptable salt thereof, such as the hydrochloride. Thus, the acyl compound may be converted into an N-protected form of (R)-1-methyl-3-phenylpropylamine, as described hereinbefore. This N-protected form may then be converted into (R)-1-hydroxy-2-[(R)-(1-methyl-3-phenylpropyl)amino] ethyl salicylamide or a pharmaceutically acceptable salt thereof according to the method of European patent application publication number 009702. Alternatively, the acyl compound may be converted into the free amine, and reacted directly with the O-protected 4-hydroxy-alpha-bromo- 3-carbamoylacetophenone, or a reactive equivalent thereof.

The N-acyl-1-methyl-omega-phenylalkylamine used as starting material in the process according to the invention may readily be prepared by acylating the corresponding 1-methyl-omega-phenylalkylamine using conventional techniques. For example, the amine may be reacted with an appropriate acyl halide or acid anhydride.

The following examples illustrate the invention.

The media used were as follows:-

| JCM Medium | |
|---|---|
| Quantities in g/l | |
| $K_2HPO_4$ | 2 |
| $MgSO_4.7H_2O$ | 0.2 |
| $FeSO_4.7H_2O$ | 0.0025 |
| $CaCl_2.2H_2O$ | 0.0125 |
| $ZnSO_4.7H_2O$ | 0.0025 |
| $MnSO_4.3H_2O$ | 0.0025 |
| $(NH_4)_2SO_4$ | 1 |
| Glucose | 10 |
| Bacto peptone | 5 |
| Yeast extract | 3 |
| Malt extract | 3 |
| pH adjusted to 7.2 | |

| Ammonium-Free Mod D2 | |
|---|---|
| Quantities in g/l | |
| $Na_2HPO_4$ | 3 |
| $KH_2PO_4$ | 3 |
| $MgSO_4.7H_2O$ | 0.2 |
| $CaCl_2.2H_2O$ | 0.0147 |
| $FeCl_3$ | 0.0167 |
| $ZnSO_4.7H_2O$ | 0.00036 |
| $CuSO_4.5H_2O$ | 0.00032 |
| $MnSO_4.4H_2O$ | 0.0003 |
| $CoCl_2.6H_2O$ | 0.00036 |
| $H_3BO_3$ | 0.00002 |
| $Na_2MoO_4.2H_2O$ | 0.0006 |
| pH adjusted to 6.8 | |

To this medium, 0.5 ml/l of the following vitamin mixture was added (quantities in mg/l):

| Biotin | 40 |
|---|---|
| Folic acid | 40 |
| Pyridoxine.HCl | 200 |
| Riboflavin | 10 |
| Thiamine | 10 |
| Nicotinic acid | 100 |
| Ca. pantothenate | 100 |
| Vitamin B12 | 2 |
| P-Aminobenzoic acid | 100 |
| Thioctic acid | 100 |

| PSX | |
|---|---|
| Quantities in g/l | |
| $KH_2PO_4$ | 8.92 |
| $Na_2HPO_4$ | 2.84 |
| $(NH_4)_2HPO_4$ | 1 |
| $(NH_4)_2SO_4$ | 0.2 |
| KCl | 0.2 |
| Trisodium citrate | 0.294 |
| $CaSO_4.7H_2O$ | 0.005 |
| $MgSO_4.7H_2O$ | 0.2 |
| $ZnSO_4.7H_2O$ | 0.0005 |
| $MnCl_2.4H_2O$ | 0.0003 |
| $CuSO_4.5H_2O$ | 0.00015 |
| $H_3BO_3$ | 0.00005 |
| $Na_2MoO_4.2H_2O$ | 0.000055 |
| KI | 0.0001 |
| $(NH_4)_2SO_4.FeSO_4.6H_2O$ | 0.0025 |
| pH adjusted to 7.0 | |

Example 1

Preparation of (R)-N-acetyl-1-methyl-3-phenylpropylamine from (RS)-N-acetyl-1-methyl-3-phenylpropylamine using Rhodococcus sp. NCIMB 40139 as biocatalyst

Rhodococcus sp. NCIMB 40139 was grown and stored on nutrient agar slopes. A slope wa⸱ ⸱⸱d to inoculate a 250 ml conical flask containing 100 ml of sterile JCM medium. The flask was incubated in an orbital incubator (to maintain aerobic conditions) for 48 h at 30° C.

The cells were precipitated from the growth medium by centrifugation and resuspended in 20ml 0.1 M potassium phosphate pH 7.O. (RS)-N-acetyl-1-methyl-3-phenylpropylamine (100mg) was added. The cell suspension and substrate in a 250 ml conical flask were incubated at 30° C in an orbital shaker for 48 h.

The pH of the cell suspension was adjusted to 11 using 2 M NaOH. The suspension was extracted with 20 ml dichloromethane. The amounts of N-acetyl-1-methyl-3-phenylpropylamine and 1-methyl-3-phenylpropylamine recovered were determined by gas chromatography under the following conditions:-

Instrument: Varian 3700 gas chromatograph

Column: 5% JXR on Gas Chrom Q (100-120 mesh), 1.84 m x 2 mm i.d. (Phase Separations, Queensferry, Clwyd, UK)

Carrier gas: $N_2$ at 30 ml/min

Injector: 200° C

Detector: Flame ionisation detector, 220° C

Oven: Column initially at 140° C (2 min) then programmed at 10° C/min to 190° C.

A calibration plot using authentic standards was prepared.

According to this method, it was found that 2.51 g/l N-acetyl-1-methyl-3-phenylpropylamine and 1.12 g/l 1-methyl-3-phenylpropylamine had been present in the cell suspension at the end of the incubation. N-acetyl-1-methyl-3-phenylpropylamine and its hydrolytic product 1-methyl-3-phenylpropylamine were purified by differential acid/base/solvent extraction. The enantiomeric composition of the two compounds was determined by resolving the enantiomers on a chiral HPLC column:

Instrument: Gilson isocratic system

Detector: UV at 254 nm

Column: Chiralcel 0D 250 mm x 4.6 mm i.d. (supplied by Hichrom, Reading, Berkshire, UK)

Mobile phase: Hexane/propane-2-ol/diethylamine (80:20:0.1)

Flow rate: 1 ml/min for analysing 1-methyl-3-phenylpropylamine or 2 ml/min for analysing N-acetyl-1-methyl-3-phenylpropylamine

Temperature: Ambient
Retention Time:
1-methyl-3-phenylpropylamine
R enantiomer approx. 3.1 min
S enantiomer approx 3.8 min
N-acetyl-1-methyl-3- phenylpropylamine
R enantiomer approx. 5.5 min
S enantiomer approx. 6.5 min

Both of the samples tested were found to contain a single enantiomer: in the case of the N-acetyl-1-methyl-3-phenylpropylamine this was the R enantiomer (> 99.5% R) whereas the 1-methyl-3-phenylpropylamine was the S enantiomer (> 99.5% S). In this and subsequent examples the stated enantiomeric purity of the R enantiomer is defined as 100% [R enantiomer]/([R enantiomer] + [S enantiomer]). Similarly the enantiomeric purity of the S enantiomer is defined as 100% [S enantiomer]/ ([R enantiomer] + [S enantiomer]).

Evidence for the order of elution of the enantiomers was obtained as follows.

In the case of the sample of 1-methyl-3-phenylpropylamine produced by the hydrolysis of (RS)-N-acetyl-1-methyl-3-phenylpropylamine by Rhodococcus sp. NCIMB 40139, the peak corresponded to the slower-eluting enantiomer (approx. retention time 3.8 min). The compound was found to be dextrotatory with $[\alpha]D^{25} = +3.4°$ (c = 0.6, CHCl$_3$). It is known from the literature that the S enantiomer of 1-methyl-3-phenylpropylamine is dextrotatory (for example $[\alpha]D^{20} = +10°$ (neat) was reported by Yamamoto, Y., Oda, J. and Inouye, Y. (1975) Bull Chem. Soc. Japan 48, 3744).

Further evidence for the slower-eluting enantiomer of 1-methyl-3-phenylpropylamine having the S configuration was obtained as follows.

A small quantity of an acicular (-)-mandelic acid salt of 1-methyl-3-phenylpropylamine was prepared by fractional crystallisation. The free base liberated from this salt was the pure, slower-running enantiomer on the chiral HPLC column. To determine the absolute configuration of this enantiomer, the circular dichroism of the benzylidene derivative was measured, as follows.

The acicular amine (-)-mandelate (3 mg, 10 µmol), 2,2,4-trimethylpentane (0.5 ml) and 2 M aqueous NaOH (4 drops) were shaken under N$_2$. Most of the upper layer was separated and heated at 80°C for 1 h with benzaldehyde (2 µmol) and two pellets of Linde 4A molecular sieve. Gas chromatographic analysis (25 m CpSil 5; 90-250°C at 10°C/min) showed the presence of benzaldehyde (retention index 950; 52%), 1-methyl-3-phenylpropylamine (r.i. 1225; 0.3%), and the benzylidene derivative (r.i. 1890; 47%). This solution, diluted 800 times, was used to measure the c.d. spectrum, which showed a broad positive maximum at 242 nm, delta epsilon + 10 (based on the mass of amine mandelate). For the benzylidene derivative of (S)-(+)-1-methyl-3-phenylpropylamine in 2,2,4-trimethylpentane, Potapov et al found delta epsilon $_{max}$ + 11.5 at 243 nm, corrected for the optical purity of the amine (Potapov, V.M., Dem'yanovich, V.M., Solov'eva, L.D., and Vendrova, O.E. (1978) Zh. Org. Khim 14,882-883). Therefore the S amine is the slower-running enantiomer on the chiral HPLC column.

The absolute configuration of the N-acetyl-1-methyl-3-phenylpropylamine being R was inferred from the fact that (S)-1-methyl-3-phenylpropylamine was the hydrolytic product. On this basis, the R enantiomer of N-acetyl-1-methyl-3-phenylpropylamine is the faster eluting enantiomer from the chiral HPLC column.

Example 2

Preparation of (R)-N-acetyl-1-methyl-3-phenylpropylamine from (RS)-N-acetyl-1-methyl-3-phenylpropylamine using Corynebacterium pseudodiphtheriticum NCIMB 40145 as biocatalyst.

Corynebacterium pseudodiphtheriticum NCIMB 40145 was grown and stored on nutrient agar slopes. The procedure of Example 1 was repeated except that the bacterium was grown in ammonium-free Mod D2 medium supplemented with glucose (5 g/l) and acetonitrile (0.5 g/l). The incubation with (RS)-N-acetyl-1-methyl-3-phenylpropylamine was for 100 h.

At the end of the incubation, the cell suspension was found to contain 2.54 g/l N-acetyl-1-methyl-3-phenylpropylamine and 2.16 g/l 1-methyl-3-phenylpropylamine. The N-acetyl-1-methyl-3-phenylpropylamine contained > 99.5% of the R enantiomer.

## Example 3

Isolation of bacteria capable of utilising N-sec-butylacetamide as carbon source

Organisms capable of utilising N-sec-butylacetamide as carbon source for growth were isolated in the following way.

To 50 ml PSX medium in 250 ml conical flasks was added 1 g of soil plus 0.25 g (5 g/l) N-sec-butylacetamide. The mixtures were incubated at 30°C in an orbital incubator for 3 to 7 days. At intervals 1 ml aliquots were removed from the flasks and used to inoculate fresh flasks containing 50 ml PSX plus 5 g/l N-sec-butylacetamide. This subculturing procedure was carried out twice after which a sample of culture was plated onto nutrient agar. The agar plates were incubated at 30°C for 1 to 3 days, following which single colonies were picked off the surface of the plate. The pure cultures obtained in this way were grown (at 30°C) and stored (at 4°C) on nutrient agar slopes.

Isolates obtained in this way were identified using the API 20B test kit (API-bioMeriux, Basingstoke, Hampshire, U.K.) as being Arthrobacter spp, and Corynebacterium spp.

## Examples 4-15

Preparation of (R)-N-acetyl-1-methyl-3-phenylpropylamine from (RS)-N-acetyl-1-methyl-3-phenylpropylamine using bacteria isolated on N-sec-butylacetamide as carbon source

Twelve bacterial isolates (designated A to L), isolated as described in Example 3, were grown as follows. Nutrient agar slopes of each isolate were used to inoculate 250 ml conical flasks containing 100 ml PSX medium and 5 g/l N-sec-butylacetamide. The flasks were incubated in an orbital incubator (to maintain aerobic conditions) for 48 to 72 h.

Cells grown in this way were incubated with (RS)-N-acetyl-1-methyl-3-phenylpropylamine (5 g/l) as described in Example 1 for 6 or 24 h. At the end of the incubation, the remaining N-acetyl-1-methyl-3-phenylpropylamine and 1-methyl-3-phenylpropylamine were extracted from the cell suspension as before.

The results are shown in Table 1. In all cases the N-acetyl-1-methyl-3-phenylpropylamine recovered was in the R form.

## Example 16

Preparation of (R)-N-acetyl-1-methyl-3-phenylpropylamine from (RS)-N-acetyl-1-methyl-3-phenylpropylamine using Arthrobacter sp NCIMB 40140 grown on lactate

Arthrobacter sp. NCIMB 40140 ("Isolate A") was grown as in Example 4, except that sodium lactate (10 g/l) was substituted for N-sec-butylacetamide as growth substrate. Acetanilide (0.5 g/l) was also added. Growth was at 30°C for 48 h.

Cells grown in this way were incubated with (RS)-N-acetyl-1-methyl-3-phenylpropylamine (at 10 g/1) as in Example 1 for 4.5 h. At the end of the incubation the cell suspension was basified (to pH 11) with 2 M NaOH. The suspension was extracted twice with 20 ml dichloromethane. The combined dichloromethane extracts were analysed for N-acetyl-1-methyl-3-phenylpropylamine and 1-methyl-3-phenylpropylamine by gas chromatography. This showed that the cell suspension had contained 4.75 g/l N-acetyl-1-methyl-3-phenylpropylamine and 4.2 g/1 1-methyl-3-phenylpropylamine. The purified N-acetyl-1-methyl-3-phenylpropylamine was found to contain the single R enantiomer (> 99.5%R) when analysed by chiral HPLC.

## Example 17

Hydrolysis of (R)-N-acetyl-1-methyl-3-phenylpropylamine

A sample (840 mg) of (R)-N-acetyl-1-methyl-3-phenylpropylamine (prepared by resolving the racemate using Arthrobacter sp. NCIMB 40140 as described in Example 4) was heated with aqueous 1.7 M sulphuric acid (9 ml) under reflux for 48 h. The mixture was cooled, diluted with water (5 ml) and extracted four times with diethyl ether. The aqueous layer was brought to pH 13 using aqueous 10 M potassium hydroxide (this and subsequent operations were carried out under nitrogen) and extracted three times with diethyl ether. The ethereal extract was dried over anhydrous magnesium sulphate and evaporated, and the residue was distilled at 70 torr (bath 130-150°C). (R)-1-methyl-3-phenylpropylamine was obtained as a colourless liquid, $[\alpha]$ D$^{20}$ = -1.6° (c = 2.2, methanol). The g.l.c. retention index (25 m Chrompack CpSil5 capillary column) and infra red spectrum (liquid film) were identical with those for (RS)-1-methyl-3-phenylpropylamine.

The absolute stereoconfiguration of the resulting 1-methyl-3-phenylpropylamine being R was confirmed by preparing the benzylidene derivative and measuring its circular dichroism in the following way. The redistilled 1-methyl-3-phenylpropylamine (5.0 μl, 30.9 μmol), benzaldehyde (5 μl, 49 μmol) and 2,2,4-trimethylpentane (0.2 ml) were warmed at 60°C for 20 minutes. G.l.c. analysis (25 m Chrompack CpSil5, 90-250°C at 10°C/min) indicated complete conversion of the amine into N-benzylidene-1-methyl-3-phenylpropylamine (retention index 1890). The mixture was made up to 50 ml with 2,2,4-trimethylpentane and further diluted 20 times for the circular dichroism measurement, which gave delta epsilon $_{max}$-11.7 at 241.5 nm. This can be compared to a value of +11.5 at 243 nm for the benzylidene derivative of (S)-1-methyl-3-phenylpropylamine in 2,2,4 trimethylpentane, corrected for optical purity (Potapov, V.M. Dem'yanovich, V.M., Solov'eva, L.D. and Vendrova, O.E. (1978) Zh.Org. Khim 14,882-883).

The enantiomeric purity of the (R)-1-methyl-3-phenylpropylamine was confirmed by Chiral HPLC as described in Example 1. Using this technique it was found that the sample contained >99.5% of the R enantiomer.

Further confirmation of the enantiomeric purity of the (R)-1-methyl-3-phenylpropylamine was obtained in the following way. 1.5 mg of 1-methyl-3-phenylpropylamine and 5 mg of (+)-10-camphorsulphonyl chloride (Fluka) were added to 0.1 ml diethyl ether. After leaving for 15 min at room temperature, 1 ml of 0.5 M aqueous sulphuric acid was added. The (+)-10-camphorsulphonamide derivative was extracted into 2 ml diethyl ether. The ethereal extract was decanted and the solvent evaporated. The sample was subjected to the following HPLC analysis:

Column: Spherisorb-NH$_2$, 250 mm x 4.6 mm i.d., 5 m particle size (Hichrom, Reading, Berkshire, UK)
Mobil phase: Hexane/dichloromethane/propan-2-ol (240:9:1) at 4 ml/min
Detector: UV at 254 nm
Temperature: Ambient

The (+)-10-camphorsulphonamide diastereomeric derivatives of (RS)-1-methyl-3-phenylpropylamine had retention times of 15.0 and 17.8 min. By contrast the (+)-10-camphorsulphonamide of the (R)-1-methyl-3-phenylpropylamine ran as a single peak with retention time 15.1 min - no trace of the diastereoisomer corresponding to the S enantiomer was observed, indicating that >99.5% of the R enantiomer was present.

Example 18

Preparation of (R)-N-acetyl-1-methyl-3-phenylpropylamine from (RS)-N-acetyl-1-methyl-3-phenylpropylamine using Arthrobacter sp NCIMB 40140 grown on nutrient broth in the presence of thioacetanilide

Arthrobacter sp NCIMB 40140 ("Isolate A") was grown as in Example 1, except that the medium used was nutrient broth (13 g/l; supplied by Oxoid Ltd., Basingstoke, Hampshire, UK). Thioacetanilide (0.25 g/l) was also added. Growth was at 30°C for 48 h.

Cells grown in this way were incubated with (RS)-N-acetyl-1-methyl-3-phenylpropylamine (at 10 g/l) as in Example 1 for 4.5 h. The cells were removed by centrifugation and the supernatant acidified (to pH 2) with 1N H$_2$SO$_4$. The supernatant was then extracted with 20 ml dichloromethane. The dichloromethane extract was washed twice with 10 ml of 1N H$_2$SO$_4$. Analysis of the dichloromethane extract showed that no 1-methyl-3-phenylpropylamine was present, only N-acetyl-1-methyl-3-phenylpropylamine. After removing the dichloromethane by rotary evaporation, the N-acetyl-1-methyl-3-phenylpropylamine was examined by chiral HPLC as described in Example 1. It was found to contain the single R enantiomer (>99.5% R).

Example 19 and 20

Preparation of (R)-N-acetyl-1-methyl-3-phenylpropylamine from (RS)-N-acetyl-1-methyl-3-phenylpropylamine using Bacillus spp NCIMB 40245 and NCIMB 40246

Bacillus spp NCIMB 40245 and NCIMB 40246 were isolated from soil as described in Example 3, except that the temperature of incubation was 37_C. The organisms were stored on nutrient agar slopes at 4_C.

The bacteria were grown in JCM medium as in Example 1, except that 5 g/l N-sec-butylacetamide was added to the growth medium and the incubation temperature was 37_C. Cells grown in this way were incubated with (RS)-N-acetyl-1-methyl-3-phenylpropylamine (10 g/l) as in Example 1, except that the temperature was 37_C. After 96 h the remaining N-acetyl-1-methyl-3- phenylpropylamine was purified as in Example 18.

Analysis by chiral HPLC showed that the N-acetyl-1-methyl-3-phenylpropylamine resulting from the incubation with Bacillus sp. NCIMB 40245 contained 76% of the R enantiomer.

The N-acetyl-1-methyl-3-phenylpropylamine resulting from the incubation with Bacillus sp. NCIMB 40246 contained 85% of the R enantiomer.

## Examples 21 and 22

Preparation of (R)-N-butyryl-1-methyl-3-phenylpropylamine and (S)-1-methyl-3-phenylpropylamine from (RS)-N-butyryl-1-methyl-3-phenylpropylamine using Arthrobacter sp. NCIMB 40140

Arthrobacter sp. NCIMB 40140 was grown in the following way.

To 100 ml sterile PSX medium in a 250 ml conical flask was added 5 g/l N-sec-butylacetamide. The organism was grown at 30_C for 48 h in an orbital incubator. Cells grown in this way were harvested by centrifugation and resuspended in 20 ml 0.1 M potassium phosphate pH 7.0 as in Example 1. To this was added (RS)-N-butyryl-1-methyl-3-phenylpropylamine (10 g/l). The mixture, in a 250 ml conical flask, was incubated at 30_C in an orbital shaker for 6 h. The remaining N-butyryl-1-methyl-3-phenylpropylamine was purified as described in Example 19.

The enantiomeric composition of the N-butyryl-1-methyl-3-phenylpropylamine was investigated by resolving the enantiomers on a Chiralcel OD HPLC column as described in Example 1 (flow rate of mobile phase 1 ml/min). According to this method the retention times of the R and S enantiomers were 5 min and 6.5 min respectively. The N-butyryl-1-methyl-3-phenylpropylamine was found to contain 99% of the R enantiomer. Further evidence for this being the R enantiomer was obtained by comparison of the circular dichroism with that of (R)-N-acetyl-1-methyl-3- phenylpropylamine. Both exhibited a positive circular dichroism in the region 195-230 nm.

In a separate incubation, the 1-methyl-3-phenylpropylamine produced as a result of hydrolysis of N-butyryl-1-methyl-3-phenylpropylamine was purified after a 3 h incubation. The enantiomeric composition of the amine was investigated by resolving the enantiomers on a Chiralcel OD HPLC column as described in Example 1 (flow rate 2 ml/min). This showed that the amine contained 91% of the S enantiomer and 9% of the R enantiomer.

## Example 23 and 24

Preparation of (R)-N-formyl-1-methyl-3-phenylpropylamine and (S)-1-methyl-3-phenylpropylamine from (RS)-N-formyl-1-methyl-3-phenylpropylamine using Arthrobacter sp. NCIMB 40140

Arthrobacter sp. NCIMB 40140 was grown and the transformation carried out as in Example 21, except that the transformation substrate was (RS)-N-formyl- 1-methyl-3-phenylpropylamine (1 g/l). The incubation was for 72 h. The remaining N-formyl-1-methyl-3-phenylpropylamine and the 1-methyl-3-phenylpropylamine produced were purified by acid/base extraction.

The enantiomeric composition of the 1-methyl-3-phenylpropylamine was investigated by resolving the enantiomers on a Chiralcel OD HPLC column as described in Example 1 (flow rate of mobile phase 2 ml/min). This method showed that the 1-methyl-3-phenylpropylamine was present as the single S enantiomer (> 99.5% S).

The enantiomeric composition of the N-formyl-1-methyl-3-phenylpropylamine was determined in the same way (flow rate 1 ml/min). According to this method, (RS)-N-formyl-1-methyl-3-phenylpropylamine was resolved into two peaks with retention times of 7.0 and 9.7 min. The microbially-derived N-formyl-1-methyl-3-phenylpropylamine was found to contain 80% of the second peak (9.7 min). Since (S)-1-methyl-3-phenylpropylamine was produced, it may be concluded that the N-formyl-1-methyl-3-phenylpropylamine was predominantly the R enantiomer (80%).

Further evidence for the N-formyl-1-methyl-3-phenylpropylamine being predominantly the R enantiomer was obtained by comparison of the circular dichroism with that of (R)-N-acetyl-1-methyl-3-phenylpropylamine. Both exhibited a positive circular dichroism in the region 200-220 nm.

## Examples 25 and 26

Preparation of (R)-N-methoxycarbonyl-1-methyl-3-phenylpropylamine and (S)-1-methyl-3-phenylpropylamine from (RS)-N-methoxycarbonyl-1-methyl-3-phenylpropylamine using Arthrobacter sp. NCIMB 40140

Arthrobacter sp. NCIMB 40140 was grown and the transformation carried out as described in Example 21, except that the transformation substrate was (RS)-N-methoxycarbonyl-1-methyl-3-phenylpropylamine. The incubation time was 48 h. Remaining N-methoxycarbonyl-1-methyl-3-phenylpropylamine and 1-methyl-3-phenylpropylamine were purified by differential acid/base/solvent extraction.

The enantiomeric composition of the 1-methyl-3-phenylpropylamine was investigated by resolving the enantiomers as in Example 1. This showed that the amine comprised 91% of the S enantiomer.

The enantiomeric composition of the N-methoxycarbonyl-1-methyl-3-phenylpropylamine was determined in a similar way. The HPLC column used was Chiralcel OB. The mobile phase contained hexane/propan-2-ol/diethylamine in the ratio 970/30/1 by volume. The flow rate was 3 ml/min and detection was by absorbance at 254 nm. Accordingly, racemic N-methoxycarbonyl-1-methyl-3-phenylpropylamine was resolved into two equal size peaks corresponding to the enantiomers with retention times of 14 and 20 min. The microbially-derived sample of N-methoxycarbonyl-1-methyl-3-phenylpropylamine was found to contain 88% of the first peak and 12% of the second. Since (S)-1-methyl-3-phenylpropylamine had been produced it may be concluded that the N-methoxycarbonyl-1-methyl-3-phenylpropylamine was predominantly the R enantiomer (88% R).

## Example 27

Preparation of (R)-N-acetyl-1-phenylethylamine from (RS)-N-acetyl-1-phenylethylamine using Arthrobacter sp. NCIMB 40140

Arthrobacter sp. NCIMB 40140 was grown as described in Example 21. Cells grown in this way were incubated with N-acetyl-1-phenylethylamine for 4 days. The remaining N-acetyl-1-phenylethylamine was purified as in Example 19.

The enantiomeric composition of the N-acetyl-1-phenylethylamine was investigated by resolving the enantiomers on a Chiralcel OD HPLC column as described in Example 1 (flow rate of mobile phase 1 ml/min). According to this method the retention times of the S and R enantiomers were 12 min and 13 min respectively. The order of elution was determined by analysis of authentic (R)-N-acetyl-1-phenylethylamine. The latter was prepared by acetylation of (R)-(+)-1-phenylethylamine (supplied by Aldrich Chemical Co., Gillingham, Dorset, UK) with acetic anhydride.

The N-acetyl-1-phenylethylamine was found to contain the single R enantiomer (>99.5% R).

## Example 28

Preparation of (S)-1-phenylethylamine from (RS)-N-acetyl-1-phenylethylamine using Arthrobacter sp. NCIMB 40140

Arthrobacter NCIMB 40140 was grown and the transformation carried out as in Example 22, except that the incubation with N-acetyl-1-phenylethylamine was for 6 h.

The enantiomeric composition of the 1-phenylethylamine produced was investigated by resolving the enantiomers on a Chiralcel OD HPLC column as described in Example 1 (flow rate of mobile phase 1 ml/min). The elution order of the enantiomers was determined by analysis of authentic (R)- and (S)-1-phenylethylamine (supplied by Aldrich Chemical Co.). The retention times were 11 and 13.5 min respectively.

The 1-phenylethylamine was found to contain the single S enantiomer (>99.5% S).

Table 1 <u>Hydrolysis of (RS)-N-acetyl-1-methyl-3-phenylpropylamine by various</u>

<u>bacteria isolated from soils which are capable of growing on N-sec-butylacetamide</u>

| Example Number | Isolate | Bacterium | NCIMB number | Source of soil | Growth time (h) | Trans- formation time (h) | Conc. Amide[2] | (g/l) Amine[3] | Enantiomeric purity of amide[2] |
|---|---|---|---|---|---|---|---|---|---|
| 4 | A | <u>Arthrobacter</u> sp. | 40140 | SH | 48 | 24 | 1.49 | 2.29 | >99.5% <u>R</u> |
| 5 | B | <u>Arthrobacter</u> sp. | 40141 | BF | 48 | 6 | 2.02 | 2.00 | >99.5% <u>R</u> |
| 6 | C | <u>Corynebacterium</u> sp. | 40142 | SH | 72 | 24 | 1.49 | 2.63 | >99.5% <u>R</u> |
| 7 | D | <u>Corynebacterium</u> sp. | 40143 | SH | 48 | 24 | 1.44 | 2.32 | >99.5% <u>R</u> |
| 8 | E | <u>Corynebacterium</u> sp. | 40144 | SH | 48 | 24 | 1.40 | 2.31 | >99.5% <u>R</u> |
| 9 | F | <u>Corynebacterium</u> sp. | - | SH | 72 | 24 | 1.43 | 2.57 | >99.5% <u>R</u> |
| 10 | G | <u>Arthrobacter</u> sp. | - | BF | 48 | 6 | 1.86 | 1.79 | >99.5% <u>R</u> |
| 11 | H | <u>Arthrobacter</u> sp. | - | BF | 48 | 6 | 1.47 | 2.06 | >99.5% <u>R</u> |

EP 0 399 589 A1

Table 1 <u>Hydrolysis of RS-N-acetyl-1-methyl-3-phenylpropylamine by various</u>

<u>bacteria isolated from soils which are capable of growing on N-sec-butylacetamide</u>

(continued)

| Example Number | Isolate | Bacterium | NCIMB number | Source of soil | Growth time (h) | Trans-formation time (h) | Conc. Amide[2] | (g/1) Amine[3] | Enantiomeric purity of amide[2] |
|---|---|---|---|---|---|---|---|---|---|
| 12 | I | <u>Arthrobacter</u> sp. | - | BF | 48 | 6 | 1.43 | 2.10 | >99.5% <u>R</u> |
| 13 | J | <u>Corynebacterium</u> sp. | - | SH | 48 | 24 | 1.42 | 2.28 | >99.5% <u>R</u> |
| 14 | K | <u>Arthrobacter</u> sp. | - | BF | 96 | 6 | 1.61 | 1.58 | >99.5% <u>R</u> |
| 15 | L | <u>Corynebacterium</u> sp. | - | BF | 96 | 6 | 1.59 | 1.58 | >99.5% <u>R</u> |

1.   Source of Soil SH − sludge farm, Shell Haven, UK

        BF − Brazilian forest soil

2.   Amide − N-acetyl-1-methyl-3-phenylpropylamine

3.   Amine − 1-methyl-3-phenylpropylamine

        Enantiomeric purity of the amide is defined as 100%x [<u>R</u>-amide]/([<u>R</u>-amide] + [<u>S</u>-amide]

EP 0 399 589 A1

## Claims

1. A process for the preparation of an N-acyl-1-methyl-omega-phenylalkylamine predominantly in the form of the R enantiomer, which comprises supplying N-acyl-1-methyl-omega-phenylalkylamine in which the acyl group is an aliphatic acyl group and the alkyl group has from 1 to 3 carbon atoms, to a biocatalyst capable of stereoselectively hydrolysing the N-acyl group of the S enantiomer, and recovering the remaining N-acyl-1-methyl-omega-phenylalkylamine predominantly in the form of the R enantiomer.

2. A process as claimed in claim 1, in which the N-acyl-1-methyl-omega-phenylalkylamine is supplied in racemic form.

3. A process as claimed in claim 1 or 2, in which the acyl group is a C(2-6) alkanoyl group, C(2-6) alkoxycarbonyl group or formyl.

4. A process as claimed in any one of claims 1 to 3, in which the biocatalyst is a microorganism, an extract of a microorganism or an enzyme.

5. A process as claimed in claim 4, in which the biocatalyst is a microorganism selected from the genera Rhodococcus, Bacillus, Arthrobacter and Corynebacterium.

6. A process as claimed in claim 4 or 5, in which the microorganism has been grown in the presence of an acetamide or a thioacetamide.

7. A process as claimed in Claim 6, in which the microorganism has been grown in the presence of thioacetanilide.

8. A process as claimed in any preceding claim, in which the alkyl group of the N-acyl-1-methyl-omega-phenylalkylamine is propyl.

9. A process as claimed in any preceding claim, in which the biocatalyst is capable of stereo-selectively hydrolysing the N-acyl group of the S enantiomer of the N-acyl-1-methyl-omega-phenylalkylamine to yield the corresponding S amine, the process further comprising the step of recovering the S amine.

10. A process as claimed in any preceding claim, further comprising the step of converting the (R)-N-acyl-1-methyl-2-phenylalkylamine into the corresponding (R)-1-methyl-2-phenylalkylamine, a salt thereof or an N-protected form thereof.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | EP-A-0 239 122 (MONTEDISON) * Claims * | 1 | C 12 P 41/00<br>C 12 P 13/00 |
| Y | EP-A-0 222 561 (MONTEDISON) * Claims * | 1 | |
| Y | PATENT ABSTRACTS OF JAPAN, vol. 13, no. 39 (C-563)[3387], 27th January 1989; & JP-A-63 237 796 (KANEGAFUCHI CHEM. IND. CO., LTD) 04-10-1988 | 1 | |
| | | | **TECHNICAL FIELDS SEARCHED (Int. Cl.5)**<br><br>C 12 P |

The present search report has been drawn up for all claims

| Place of search | Date of completion of the search | Examiner |
|---|---|---|
| THE HAGUE | 16-08-1990 | DELANGHE L.L.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

&: member of the same patent family, corresponding document

EPO FORM 1503 03.82 (P0401)